# EUROPEAN PATENT SPECIFICATION

(11) **EP 0 705 135 B1**
(45) Date of publication and mention of the grant of the patent: **27.08.1997**
(21) Application number: 94919934.3
(22) Date of filing: 16.06.1994
(51) Int. Cl.: B01F 3/12, B01F 13/06, A61F 2/46

(54) **APPARATUS FOR THE PREPARATION OF BONE CEMENT**
VORRICHTUNG ZUM HERSTELLEN VON KNOCHENZEMENT
APPAREIL DE PREPARATION DE CIMENT OSSEUX

(30) Priority: 21.06.1993 US 81445
(43) Date of publication of application: 10.04.1996
(73) Proprietor: CEMVAC SYSTEM AKTIEBOLAG, 589 41 Linköping (SE)
(72) Inventor: JONSSON, Sören, S-582 70 Linköping (SE)
(74) Representative: Willquist, Bo
(86) International application number: SE9400593
(87) International publication number: WO9500240

(56) References cited:
- WO-A-93/22041
- SE-B- 462 315

## Description

### BACKGROUND OF THE INVENTION

The present invention relates to an apparatus for manufacturing bone cement by mixing a plurality of constituent components. Such an apparatus is for example disclosed in WO-A-9013264 (SE-B-462315). WO-A-9013264 relates to an arrangement for the manufacture of bone cement by mixing together its constituent components comprising a mixing cylinder in the form of a tube with a bottom and a lid and inside the mixing cylinder an axially movable agitator in the form of a piston-like agitator disc, which is perforated by channels, through which the components of the bone cement are caused to flow during the mixing procedure and an agitator rod resembling a piston rod operatively connected to the agitator disc and supported in the lid in such a way that it is free to slide.

Bone cement is used in hip-joint operations, for example. When manufacturing bone cement, for which purpose a component in the form of a powder and a liquid component are required to be mixed, it is important that the mixture should be as homogeneous as possible, and that gases which occur during the mixing procedure are removed to the greatest extent possible. Any lack of homogeneity and gas inclusions have the effect of reducing the strength of the bone cement, which can result in a shorter service life for the implanted prothesis. Furthermore, the gases which occur during manufacture may be unhealthy, for which reasons steps must be taken to ensure that they do not escape into the operating theatre.

### SUMMARY OF THE INVENTION

The object of the present invention is to make available a preparation apparatus for bone cement, which is intended to be disposable and capable of being manufactured efficiently and at a low cost, and which also contributes to meeting the requirements stipulated above in respect of the prepared bone cement. The invention comprises an apparatus according to claim 1 for manufacturing bone cement by mixing a plurality of constituent components within a mixing cylinder. A bottom is axially removable within the cylinder, and a funnel is attachable to the cylinder for introducing the plurality of constituent components therein.

This object is met in that an evacuation channel is disposed in the funnel for drawing gas from the funnel. The evacuation channel is connectable to an evacuation source.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 illustrates an embodiment having a funnel which is removably attached to the mixing cylinder and including an evacuation channel;
Fig. 2 illustrates another embodiment having a funnel which is removably attached to the mixing cylinder, and which defines an annular space fluid communication with the evacuation channel;
Fig. 3 illustrates another embodiment having a funnel removably attached to the mixing cylinder and including an evacuation channel;
Figs. 4a and 4b illustrate another embodiment having a removable lid with an evacuation channel and a funnel having a second evacuation channel removably attached to the lid.

Corresponding reference characters indicate corresponding parts throughout the several views. The exemplification set out herein illustrates preferred embodiments of the invention and such exemplification is not to be construed as limiting the scope of the invention in any manner.

### DETAILED DESCRIPTION OF THE INVENTION

The designation 1 is used in the drawing for a mixing cylinder, and 2 for a bottom which is inserted in a sealing fashion into one end of the mixing cylinder 1. The mixing cylinder 1 is preferably made from a transparent material. The bottom 2, can be held in its position in different ways for example with a locking pin not shown or by a bayonet coupling.

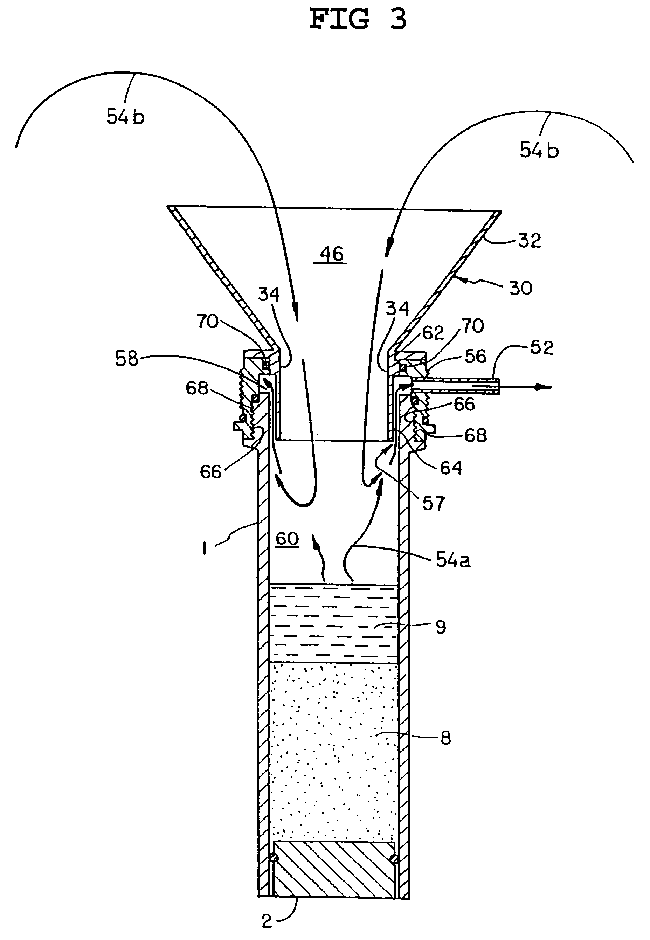

A dot screen and the designation 8 are used in the drawing to indicate a powder, and the designation 9 and a pattern of dashes to indicate a liquid, which are components of the bone cement and have been introduced into the mixing cylinder 1 via a funnel mounted to the opening which is intended to be closed off by means of a lid 3 threadingly engaged with the cylinder and integrated with an emptying tube, or as shown with an arrangement according to Fig 3. In the interests of clarity, these components are omitted from the other Figures.

The mixing procedure itself and the further use of the prepared bone cement in a syringe mechanism of the kind which is customarily used for the extrusion of sealing compound or similar is disclosed in the form of a bead and will not be explained further.

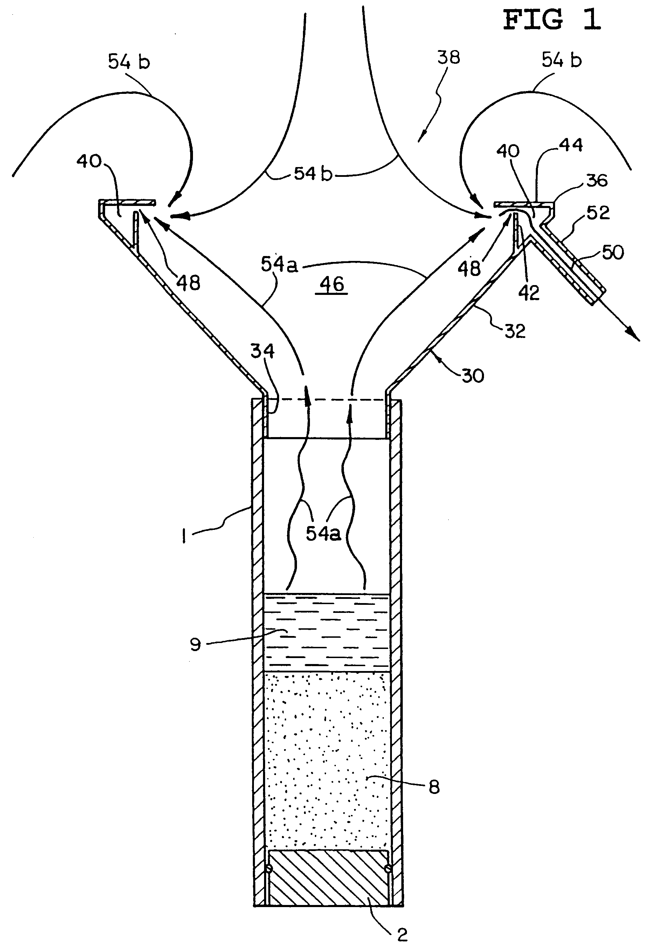

Fig. 1 illustrates an embodiment of the present invention having a funnel 30 which is removably attachable to mixing cylinder 1 for introducing a plurality of constituent components 8, 9 into mixing cylinder 1. Funnel 30 includes a frustoconical portion 32 and a smaller diameter portion 34. Smaller diameter portion 34 has an external diameter which is slightly less than the internal diameter of mixing cylinder 1. Smaller diameter portion 34 therefore may be slid within mixing cylinder 1, whereby smaller diameter portion 34 is disposed within and radially adjacent mixing cylinder 1.

Funnel 30 includes a larger diameter base portion 36 disposed at an inlet 38 of funnel 30, i.e., at the base portion of frustoconical portion 32. An air flow conduit 40, which is generally disposed at base portion 36 and defined by transversely extending walls 42 and 44, is in fluid communication with an interior 46 of funnel 30 via a gap 48, and is in further fluid communication with evacuation channel 50. Evacuation channel 50 is defined by a tube 52 attached to frustoconical portion 32, and adapted for connection to an evacuation source (not shown).

The plurality of constituent components 8, 9 may be poured into funnel 30 through inlet 38 for subsequent mixing within mixing cylinder 1. Gases produced by constituent components 8, 9 are drawn into air flow conduit 40 through gap 48 and are exhausted via evacuation channel 50. Directional arrows 54a, shown in Fig. 1, indicate the flow of gases within mixing cylinder 1 and funnel 30 which are drawn into air flow conduit 40.

Arrows 54b indicate the flow of ambient air drawn into the funnel thus forming a barrier preventing gases from escaping into the operating theatre.

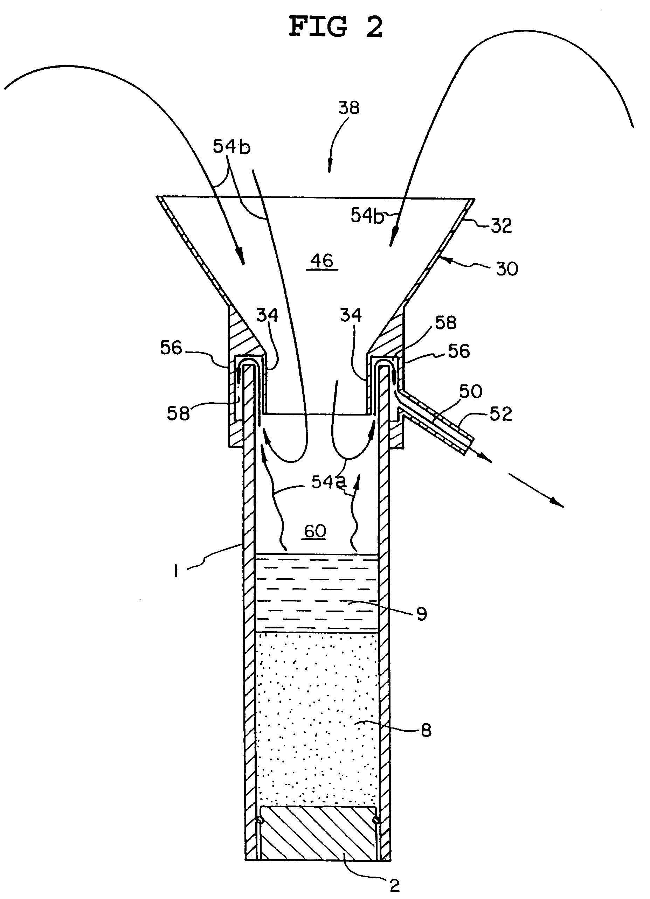

Fig. 2 illustrates another embodiment of the present invention having a funnel 30 which is removably attached to mixing cylinder 1. Funnel 30 includes a larger diameter portion 56 which is disposed radially outward from smaller diameter portion 34. Larger diameter portion 56 includes an inner diameter which is slightly larger than the exterior diameter of mixing cylinder 1 and is disposed radially adjacent mixing cylinder 1 when in an installed position. Larger diameter portion 56 and smaller diameter portion 34 define an annular space 58 therebetween which is in fluid communication with interior 60 of mixing cylinder 1 and interior 46 of funnel 30. Annular space 58 is further in fluid communication with evacuation channel 50 defined by tube 52. Gases within interior 46 of funnel 30 and interior 60 of mixing cylinder 1, as indicated by directional arrows 54a, are drawn together with ambient air 54b into annular space 58 and exhausted out evacuation channel 50.

Fig. 3 discloses another embodiment of the present invention including a funnel 30 having a frustoconical portion 32 and a smaller diameter portion 34. The exterior of smaller diameter portion 34 includes a first cylindrical portion 62 having a diameter substantially the same as the inner diameter of mixing cylinder 1, and a second cylindrical portion 64 having a diameter which is less than the inner diameter of mixing cylinder 1. Disposed radially outward from smaller diameter portion 34 is a larger diameter portion 56 having female threads 66 which threadingly engage male threads 68 formed in mixing cylinder 1. Smaller diameter portion 34 and larger diameter portion 56 define an annular space 58 therebetween.

In the embodiment shown in Fig. 3, larger diameter portion 56 is formed separately from funnel 30 and includes an O-ring 70 for sealingly engaging funnel 30. Those skilled in the art will recognize, however, that larger diameter portion 56 may be monolithically formed with funnel 30. A tube 52 defining an evacuation channel 50 is disposed in larger diameter portion 56 and is connectable to an evacuation source (not shown). Gases 54a within interior 60 of mixing cylinder 1 and interior 46 of funnel 30 are drawn together with ambient air through annular space 57 and exhausted through tube 52.

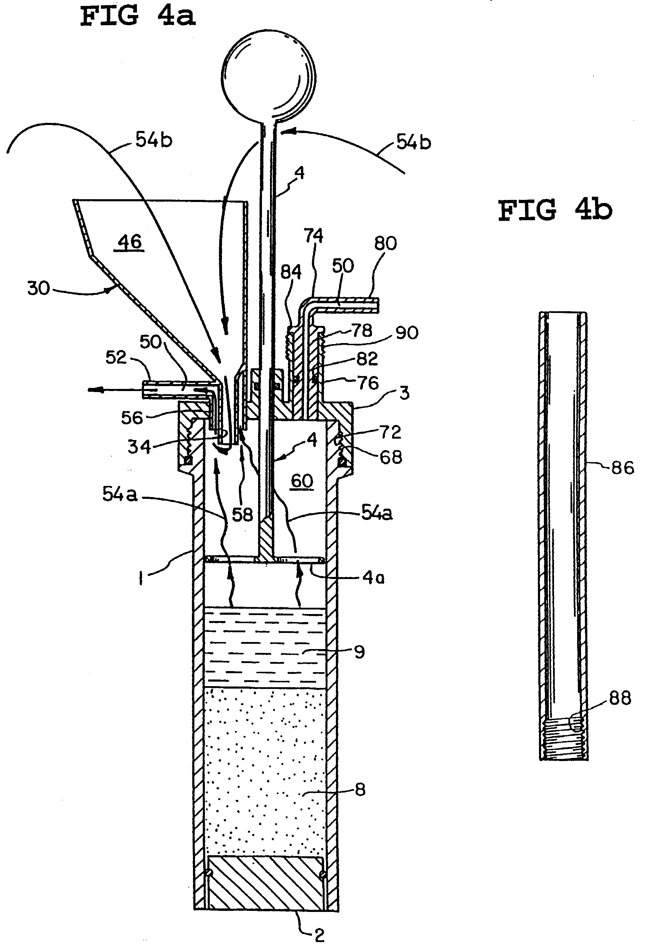

Fig. 4a illustrates another embodiment of the invention having a lid 3 with female threads 72 which engage male threads 68 formed on mixing cylinder 1. A funnel 30 having a larger diameter portion 56 and smaller diameter portion 34 defines an annular space 58 therebetween which is in fluid communication with interior 60 of mixing cylinder 1.

A tube 52 defining an evacuation channel 50 is attached to larger diameter portion 56 and in fluid communication with annular space 58. Tube 52 is adapted to be connected to an evacuation source (not shown) for drawing gases from interior 60 of mixing cylinder 1 and interior 46 of funnel 30.

The constituent components 8 and 9 are drawn into mixing cylinder 1 through funnel 30 under the influence of the negative pressure created by the evacuation of air within the cylinder. This is accomplished via annular space 58 arranged between portions 56 and 34, funnel 30 may be thereafter removed and the associated opening formed in lid 3 filled with a suitably shaped plug (not shown). To remove gases which exist within mixing cylinder 1 during the mixing process, lid 3 is formed with an upstanding tubular portion 76 adapted to receive a removable evacuation tube 74. Removable evacuation tube 74 includes a first portion 78 having an exterior diameter which is slightly less than the interior diameter of upstanding tubular portion 76, and a second portion 80 adapted for connection to a vacuum source (not shown). Removable evacuation tube 74 defines an evacuation channel 50 therein, and includes an O-ring 82 for sealingly engaging upstanding tubular portion 76. A shoulder 84 allows removable evacuation tube 74 to be seated against upstanding tubular portion 76.

After mixing of constituent components 8, 9 with agitator 4 is complete, removable evacuation tube 74 is removed from upstanding tubular portion 76 and a tube 86 (Fig. 4b), having female threads 88 is screwed onto upstanding tubular portion 76 whereby male threads 90 engage female threads 88. Bottom 2 is then moved in an axial direction toward lid 3, and the mixed constituent components 8, 9 are expelled through tube 86.

The present invention, as described above, includes a number of advantages over previously known apparatus for preparing bone cement. For example, the invention does not require premeasured components. Rather, the amount of components required can be decided in situ, i.e., in the hospital operating room.

Furthermore the type of constituent components can be selected as desired to permit total flexibility by the surgeon in selecting both the types and quantities of constituent components. In addition, by drawing gas through the cylinder and drawing the constituent components into the cylinder and forming a negative pressure at the point of entry into the cylinder for the constituent components, noxious fumes generated by the constituent components will be drawn into the cylinder and will not be admitted into the hospital operating room. This is particularly important when such fumes may be harmful to personnel in the hospital operating room and where several batches of bone cement need to be mixed within a relatively short period of time, thereby preventing unnecessary exposure of personnel to such fumes. Moreover, the present invention permits the creation of a partial vacuum within the cylinder to prevent the formation of air bubbles in the mixed bone cement, thereby preventing porosity of the bone cement and ensuring its effectiveness.

## Claims

1. An apparatus for manufacturing bone cement by mixing a plurality of constituent components, said apparatus comprising:
a mixing cylinder (1) comprising a bottom (2) which is axially movable within said cylinder;
a funnel (30) attached to said cylinder (1) for introducing said plurality of constituent components into said cylinder (1),
an evacuation channel (50) disposed in said funnel (30) for drawing gas from said funnel (30) and from said cylinder (1); and
means for connecting said evacuation channel to an evacuation source.

2. The apparatus of Claim 1, **characterized** in that said funnel (30) includes a frustoconical portion (32) and a smaller diameter portion (34), said smaller diameter portion being adapted to be disposed within and radially adjacent said cylinder (1).

3. The apparatus of claim 2, **characterized** in that said smaller diameter portion (34) engages said cylinder (1).

4. The apparatus of claim 2, **characterized** in that said funnel (30) further includes a larger diameter portion (56) disposed radially outward from said smaller diameter portion (34), said larger diameter portion (56) engaging said cylinder (1).

5. The apparatus of claim 4, **characterized** in that said larger diameter portion (56) threadingly engages said cylinder (1).

6. The apparatus of claim 4, **characterized** in that said larger diameter portion (56) includes female threads on the interior thereof, and said cylinder (1) includes male threads on the exterior thereof.

7. The apparatus of claim 4, **characterized** in that said larger diameter portion (56) and said smaller diameter portion (34) define an annular space (58) therebetween, said annular space (58) being in fluid communication with said cylinder (1) and said evacuation channel (50).

8. The apparatus of claim 4, **characterized** in that said larger diameter portion (56) forms an adapter between said funnel (30) and said cylinder.

9. The apparatus of claim 2, **characterized** in that said frustoconical portion (32) includes a larger diameter base portion disposed at the inlet to said funnel (30), and an air flow conduit (40) disposed at said larger diameter base portion, said air flow conduit (40) being in fluid communication with the interior of said funnel (30) and said evacuation channel (50).

## Patentansprüche

1. Eine Vorrichtung zur Herstellung von Knochenzement durch Mischen einer Vielzahl von Bestandteilen, wobei die Vorrichtung umfaßt:
einen Mischzylinder (1), umfassend einen Boden (2), der innerhalb des Zylinders axial beweglich ist;
einen Trichter (30), befestigt an dem Zylinder (1), für das Einführen der Vielzahl von Bestandteilen in den Zylinder (1),
einen Evakuierungskanal (50), der in dem Trichter (30) angeordnet wird, um Gas aus dem Trichter (30) und aus dem Zylinder (1) zu ziehen; und
Mittel für das Verbinden des Evakuierungskanals mit einer Evakuierungsquelle.

2. Die Vorrichtung gemäß Anspruch 1, **dadurch gekennzeichnet**, daß der Trichter (30) einen kegelstumpfförmigen Teil (32) und einen Teil mit kleinerem Durchmesser (34) einschließt, wobei der Teil mit kleineren Durchmesser daran angepaßt ist, innerhalb und radial benachbart an dem Zylinder (1) angeordnet zu werden.

3. Die Vorrichtung gemäß Anspruch 2, **dadurch gekennzeichnet,** daß der Teil mit kleineren Durchmesser (34) mit dem Zylinder (1) in Verbindung steht.

4. Die Vorrichtung gemäß Anspruch 2, **dadurch gekennzeichnet**, daß der Trichter (30) zusätzlich einen Teil mit größerem Durchmesser (56), radial außen von dem Teil mit kleineren Durchmesser (34) angeordent, einschließt, wobei der Teil mit größerem Durchmesser (56) mit dem Zylinder (1) in Verbindung steht.

5. Die Vorrichtung gemäß Anspruch 4, **dadurch gekennzeichnet**, daß der Teil mit größerem Durchmesser (56) über Gewinde mit dem Zylinder (1) in Verbindung steht.

6. Die Vorrichtung gemäß Anspruch 4, **dadurch gekennzeichnet**, daß der Teil mit größerem Durchmesser (56) Muttergewinde an seinem Inneren umfaßt und der Zylinder (1) Bolzengewinde an seinem Äußeren einschließt.

7. Die Vorrichtung gemäß Anspruch 4, **dadurch gekennzeichnet**, daß der Teil mit größerem Durchmesser (56) und der Teil mit kleinerem Durchmesser (34) einen Ringraum (58) zwischen ihnen definieren, wobei der Ringraum (58) in Fließverbindung mit dem Zylinder (1) und dem Evakuierungskanal (50) steht.

8. Die Vorrichtung gemäß Anspruch 4, **dadurch gekennzeichnet**, daß der Teil mit größerem Durchmesser (56) einen Adapter zwischen dem Trichter (30) und dem Zylinder bildet.

9. Die Vorrichtung gemäß Anspruch 2, **dadurch gekennzeichnet**, daß der kegelstumpfförmige Teil (32) einen an dem Einlaß zu dem Trichter (30) angeordneten Basisteil mit größerem Durchmesser und ein an dem Basisteil mit größerem Durchmesser angeordneten Luftstromrohr (40) einschließt, wobei das Luftstromrohr (40) in Fließverbindung mit dem Inneren des Trichters (30) und dem Evakuierungskanal (50) steht.

## Revendications

1. Appareil destiné à la fabrication d'un ciment ostéoplastique par mélange d'une pluralité de composants constitutifs, le dit appareil comprenant :
- un cylindre de mixage (1) comprenant un fond (2) qui est axialement déplaçable à l'intérieur du dit cylindre ;
- une trémie (30) reliée au dit cylindre (1) pour introduire la dite pluralité des composants constitutifs du ciment dans le dit cylindre (1),
- un ajutage (50) monté sur la dite trémie (30) pour le soutirage de fluides de la dite trémie (30) et du dit cylindre ; et
- des moyens de liaison du dit canal d'évacuation à une source de vide ;

2. Appareil selon la revendication 1, caractérisé :
en ce que la dite trémie (30) comprend une partie tronconique (32) et une partie de plus petit diamètre (34), la dite partie de plus petit diamètre étant adaptée pour être disposée à l'intérieur, et radialement adjacente, du dit cylindre (1) ;

3. Appareil selon la revendication 2, caractérise :
en ce que la dite partie de plus petit diamètre (34) s'adapte étroitement à l'intérieur du dit cylindre (1) ;

4. Appareil selon la revendication 2, caractérise :
en ce que la dite trémie (30) comprend en outre une partie de diamètre (56) plus grand disposée radialement vers l'extérieur par rapport à la dite partie de diamètre plus petit (34), la dite partie de diamètre plus grand (56) s'adaptant étroitement à l'extérieur du dit cylindre (1) ;

5. Appareil selon la revendication 4, caractérisé :
en ce que la dite partie de diamètre plus grand (56) engrène par filetage sur le dit cylindre (1);

6. Appareil selon la revendication 4, caractérisé :
en ce la partie de plus grand diamètre (56) est filetée intérieurement tandis que le dit cylindre (1) est fileté extérieurement ;

7. Appareil selon la revendication 4, caractérisé :
en ce que la dite partie de plus grand diamètre (56) et la dite partie de plus petit diamètre (34) définissent entre elles un espace annulaire (58), le dit espace annulaire (58) formant avec le dit cylindre (1) et le dit canal d'évacuation (50) un canal pour le fluide ;

8. Appareil selon la revendication 4, caractérisé
en ce que la partie de plus grand diamètre (56) forme un adaptateur entre la dite trémie(30) et le dit cylindre ;

9. Appareil selon la revendication 2, caractérise :
en ce que la dite partie tronconique (32) comprend une section de base de plus grand diamètre disposée à l'entrée de la dite trémie (30) et un conduit d'écoulement de fluide (40) disposé dans la dite section de base de plus grand diamètre, le dit conduit d'écoulement (40) étant en communication fluidique avec l'intérieur de la dite trémie (30) et avec le dit canal d'évacuation (50).
